# EUROPEAN PATENT APPLICATION

(11) **EP 4 091 545 A1**
(43) Date of publication of application: **23.11.2022**
(21) Application number: 21174984.1
(22) Date of filing: 20.05.2021
(51) Int. Cl.: A61B 5/24, A61B 5/00, A61B 5/024

(54) **ELECTRODE CONFIGURATION FOR ELECTROPHYSIOLOGICAL MEASUREMENTS**

(71) Applicant: Koninklijke Philips N.V., 5656 AG Eindhoven (NL)
(72) Inventor: MISCHI, Massimo, 5656 AE Eindhoven (NL); RABOTTI, Chiara, 5656 AE Eindhoven (NL); PERI, Elisabetta, 5656 AE Eindhoven (NL); GALLI, Alessandra, 5656 AE Eindhoven (NL)
(74) Representative: Philips Intellectual Property & Standards

(57) **Abstract**

Proposed are concepts for generating and using a prediction model that enables the prediction of the quality (e.g. accuracy and/or reliability) of an electrode configuration (i.e. arrangement) for electrophysiological measurements. It is proposed to use training data to determine parameter values of a prediction model so that the prediction model is configured to accurately predict a quality value (e.g. measure of accuracy) of an arrangement or pattern of electrodes for electrophysiological measurements of a subject. Embodiments may thus facilitate the assessment of an electrode configuration and/or the recommendation of changes to an electrode configuration so as to enable more reliable and/or accurate electrophysiological measurements to be obtained.

## Description

### FIELD OF THE INVENTION

The invention relates to the field of electrophysiological measurements of a subject and more particularly to the configuration of electrodes for electrophysiological measurements.

### BACKGROUND OF THE INVENTION

Electrophysiological measurements are typically acquired via an array of electrodes placed on a region of a subject's body (i.e. patient). The signals acquired by such electrodes consists of a mixture of (unwanted) signals and noise sources superimposed on an electrophysiological signal of interest (e.g. electrocardiogram, ECG). The signal of interest may extracted (i.e. separated from the noise and unwanted signal(s)) by dedicated signal processing strategies, so as to facilitate/support clinical interpretation for example.

Electrophysiological measurements have been demonstrated to be more accurate than standard diagnostics and may facilitate further benefits (such as enabling long term remote monitoring).

Furthermore, electrophysiological measurements are less sensitive to the exact positioning of the electrodes for reliable measurements. However, an ECG signal, for example, may be weak compared to the noise with which it is captured. This can make extraction of the signal of interest difficult. Accordingly, optimal positioning of electrodes can affect the accuracy and/or reliability of acquired electrophysiological measurements.

### SUMMARY OF THE INVENTION

The invention is defined by the claims.

In accordance with one aspect, there is provided a method of generating a prediction model configured to predict a quality value of an electrode configuration (i.e. positional arrangement or relative positioning of the electrodes) for electrophysiological measurements, the method comprising: inputting a plurality of training electrode signals for a plurality of known electrode configurations, and with a plurality of known quality values to the prediction model;
receiving a plurality of predicted quality values from the prediction model; and
determining parameter values of the prediction model based on a difference between the plurality of known quality values and the plurality of predicted quality values.

By way of example, proposed concepts may provide a prediction model that enables the prediction of the quality (e.g. accuracy and/or reliability) of an electrode configuration (i.e. arrangement) for electrophysiological measurements. That is, embodiments propose to use training data to determine parameter values of a prediction model, so that the prediction model is configured to accurately predict a quality value (e.g. measure of accuracy) of an arrangement or pattern of electrodes for electrophysiological measurements of a subject. In this way, embodiments may facilitate the assessment of an electrode configuration and/or the recommendation of changes to an electrode configuration so as to enable more reliable and/or accurate electrophysiological measurements to be obtained.

Reference to an electrode configuration is to be taken to refer to the positional arrangement, pattern or relative placement of a plurality of electrodes. Thus, the electrodes of a first electrode configuration may be positioned relative to each other (e.g. spaced apart from each other by the same or varying distances) on a subject so as to be in a first set of positions. The electrodes of a second, different electrode configuration may then be positioned relative to each other on a subject so as to be in a second, different set of positions. In this way, first and second different electrode configurations comprise different positional arrangements of electrodes (i.e. different relative placements or positions of electrodes).

By way of example, proposed embodiments may provide a model which assists in determining the an optimal electrode location for fetal heart rate (fHR) extraction (which is particularly relevant antepartum (i.e. before delivery) when the fetal position can frequently change). Although approaches to investigating optimal electrode positioning for fHR monitoring are known (e.g. using results of ECG analysis), no consensus has been reached on a unique preferred configuration. Proposed embodiments, however, may support the determination of optimal electrode configuration for fetal ECG acquisition and analysis. In particular, based on the raw signals from multichannel recordings, the quality of a fetal ECG acquisition using an electrode configuration may be predicted prior to signal acquisition. This may enable the optimal use of small electrode sets, as well as the automatic selection of optimal electrode subsets from larger electrode grids. In particular, some embodiments may be based on an understanding of blind source separation (BSS) techniques that are typically employed for fetal ECG analysis.

Optimal electrode configurations (i.e. positioning or placement of electrodes) may minimize a number of components required to represent noise and interference sources, thus enabling high quality signal measurements with a reduced number of electrodes. Indeed, the use of fewer electrodes may facilitate the implementation of continuous monitoring (e.g. by reducing power consumption, reduced complexity of electronic design, and/or reducing a required data capacity and/or bandwidth).

A prediction model generated in accordance with a proposed embodiment may enable automatic assessment of the quality of an electrode configuration for acquiring and/or monitoring electrophysiological measurements (such as fECG in a pregnant woman). In particular, use of a prediction model according to an embodiment may assist in: (i) the identification of an optimal electrode configuration among a set of available configurations; or (ii) the determination of whether a particular electrode configuration meets a required quality measure (e.g. whether an electrode configuration is accurate enough for signal analysis and interpretation). Based on a multichannel recording system, an outcome of such an automatic assessment may be determined via analysis of raw signal data, without need for any pre-processing of the signals and/or without performing resource intensive processing for signal extraction and/or analysis.

The employed electrodes may be of any type: contact Ag-AgCl, capacitive, or dry electrodes. As the most advanced and accurate algorithms for analysis of the fetal ECG are based on BSS techniques (such as principal component analysis, independent component analysis, and singular value decomposition), the embodiments may support assessment of electrode configuration quality through the evaluation of a number of signal features that reflect the linear and nonlinear correlation between the channels.

Proposed is the provision of a prediction model that is able to predict the quality of an electrode configuration for electrophysiological measurements (such as such as electrocardiography, electromyography, electrogastrography or electrohysterography for example). Such a model may be used to determine whether an electrode configuration is sufficiently good for accurate or reliable signal acquisition and analysis. A prediction from such a model may be based on selected features extracted from the raw signals.

In some embodiments, the prediction model may use a classifier or regressor. For instance, the prediction model may employ a support vector machine (e.g. for classification) and/or support vector regression (e.g. for regression), with the latter providing a quality score that can again be used for classification and decision making by thresholding. Lasso or Bridge regression, gaussian mixture models, or K-nearest neighbours may also be employed, as to many other neural network architectures. Such approaches allow for the determination of a confidence level that may assist decision making.

The plurality of training electrode signals may comprise training electrode signals with the one or more signal variables. The prediction model may then be configured to represent an interaction between with the one or more signal variables and the plurality of known quality values. By way of example, the one or more signal variables may include kurtosis of channels, skewness of channels, variance of channels, correlation between channels, mutual Information between channels, spectral coherence between channels, entropy of channels, or cross-entropy between channels.

In some embodiments, the quality value may comprise at least one of: an F-score; root mean squared error; area under the receiver operating characteristic curve; accuracy; sensitivity; specificity; positive predictive value; and correlation coefficient. Various measures of quality may therefore be employed by embodiments, thus enabling improved understanding and/or accuracy of electrode configuration performance (e.g. via the use of multiple and/or different quality measures).

Determining parameter values of the prediction model may comprise: adjusting parameter values of the prediction model so as to decrease a difference between: a known quality value associated with training electrode signals for a known electrode configuration; and a predicted quality value received from the prediction model for the known electrode configuration. Relatively simple, iterative-based training/learning concepts may thus be employed by proposed embodiments. This may, in turn, reduce the cost and/or complexity of implementation.

According to another aspect of the invention, there is provided a method for processing electrode signals from an electrode configuration for electrophysiological measurements, the method comprising: generating a prediction model according to a proposed embodiment; obtaining electrode signals from an electrode configuration for obtaining electrophysiological measurements of a subject; and obtaining a prediction of a quality value of the electrode configuration based on inputting the electrode signals to the generated prediction model.

Some embodiments may further comprise identifying a modification to the electrode configuration based on the predicted quality value of the electrode configuration. Embodiments may therefore facilitate dynamic modification and/or optimization of an electrode configuration.

In some embodiments, the method may further comprise classifying the electrode configuration into one of a plurality of classifications based on the predicted quality value of the electrode configuration. For example, classifying the electrode configuration may comprise: comparing the predicted quality value of the electrode configuration against at least one threshold value; and classifying the electrode configuration into one of a plurality of classifications based on the comparison result. Relatively simple, quality assessment concepts may thus be employed by proposed embodiments. This may, in turn, reduce the cost and/or complexity of implementations.

According to yet another aspect of the invention, there is provided a method for processing electrode signals from an electrode configuration for electrophysiological measurements using a prediction model configured to predict a quality value of an electrode configuration, the method comprising: obtaining electrode signals from an electrode configuration for obtaining electrophysiological measurements of a subject; and obtaining a prediction of a quality value of the electrode configuration based on inputting the electrode signals to the generated prediction model.

The quality value may comprise at least one of: an F-score; root mean squared error; area under the receiver operating characteristic curve; accuracy; sensitivity; specificity; positive predictive value; and correlation coefficient.

An embodiment may further comprise: identifying a modification to the electrode configuration based on the predicted quality value of the electrode configuration.

According to another aspect of the invention, there is provided a computer program product comprising computer program code means which, when executed on a computing device having a processing system, cause the processing system to perform all of the steps of the method according to a proposed embodiment. Thus, according to examples in accordance with an aspect of the invention, there is provided a computer program comprising code means for implementing the method according to a proposed embodiment when said program is run on a processing system.

According to yet another aspect of the invention, there is provided a system for processing electrode signals from an electrode configuration for electrophysiological measurements using a prediction model configured to predict a quality value of an electrode configuration, the system comprising: a modelling unit configured to generate a prediction model configured to predict a quality value of an electrode configuration for electrophysiological measurements; an interface configured to obtain electrode signals from an electrode configuration; and a processing unit configured to acquire a prediction of a quality value of the electrode configuration based on inputting the electrode signals to the generated prediction model.

Proposed embodiments may provide and/or use a prediction model configured to predict a quality value of an electrode configuration for electrophysiological measurements. Embodiments may therefore be of particular use/benefit in combination with any medical device and/or procedure dealing with multichannel surface biopotential measurements. Purely by way of example, embodiments may be particularly beneficial for fetal monitoring. However, embodiments may be applicable other subject monitoring applications which rely on electrophysiological measurements.

Still further advantages of the present invention will be appreciated to those of ordinary skill in the art upon reading and understand the following detailed description.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 simplified flow diagram of a method for generating a prediction model according to an embodiment;
Fig. 2 is a is a simplified flow diagram of a method for processing electrode signals from an electrode configuration for electrophysiological measurements according to an embodiment;
Figs. 3A-3C are graphs illustrating the performance of SVM classification in terms of sensitivity, specificity, and F-score of the class (good or poor configuration) prediction for increasing number of features, using either the F-score or the RMSE as separate indices (Figs. 3A & 3B) or their combination (Fig. 3C);
Figs. 4A-4C are graphs illustrating the performance of SVR prediction of the resulting F-score and RMSE separately (Figs. 4A & 4B) or their combination (Fig. 4C);
Fig. 5 illustrates preferred electrode configurations for cephalic and podalic presentation of the fetus using five electrodes (as predicted by a prediction model according to an embodiment);
Fig. 6 illustrates an exemplary electrode grid denoting a set of sixteen possible electrode positions;
Fig. 7 illustrates eight different 5-electrode configurations for the electrode grid of Fig. 6; and
Fig. 8 is simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts for model and/or predicting the quality (e.g. accuracy and/or reliability) of an electrode configuration (i.e. arrangement) for electrophysiological measurements.

For instance, embodiments may provide a prediction model that is configured to assess the quality of a certain electrode configuration. Based on such an assessment, it may be determined whether the electrode configuration meets one or more predetermined requirements for reliable signal acquisition and/or analysis. The model may employ one or more features extracted from the raw signal(s) of the electrodes.

The proposed prediction model may be implemented in several ways. For example, a support vector machine (SVM) may be employed for classification and a support vector regression (SVR) may be used for regression. Such regression may, for example, determine a quality score for an electrode configuration, and such a quality score may be used classification and/or decision making (e.g. by comparison against one or more threshold values).

Embodiments may support or facilitate the determination of an optimal electrode configuration (i.e. spatial arrangement) with respect to one or more particular requirements. For instance, embodiments may be used to identify an optimal electrode configuration for fetal ECG acquisition. Using raw signals from multichannel recordings, the quality of planned/subsequent electrophysiological measurements may be predicted prior to acquisition. In particular, embodiments may enable the identification of a best/optimal electrode configuration from a set of available electrode configurations. Additionally, or alternatively, embodiments may determine whether an electrode configuration is good enough (e.g. meets accuracy and/or reliability requirements) for signal acquisition.

A large set of features has been tested and the prediction models employed for feature selection.

Proposed embodiments may therefore be employed to provide electrode configuration recommendations that help to improve the accuracy and/or reliability of electrophysiological measurements.

Embodiments may, for example, facilitate generation of personalized/customized recommendations for electrode placement/arrangement that are tailored/adapted to specific circumstances and/or requirements. For example, a prediction model according to proposed embodiments may be used to provide refined electrode configuration recommendations.

Illustrative embodiments may, for example, be employed in relation to healthcare-related services, including the acquisition and/or analysis of electrophysiological measurements.

Implementations in accordance with the present disclosure relate to various systems, adaptions, and/or methods for predicting or assessing a quality value of an electrode configuration for electrophysiological measurements. According to proposed concepts, a number of possible solutions may be implemented separately or jointly. That is, although these possible solutions may be described below separately, two or more of these possible solutions may be implemented in one combination or another.

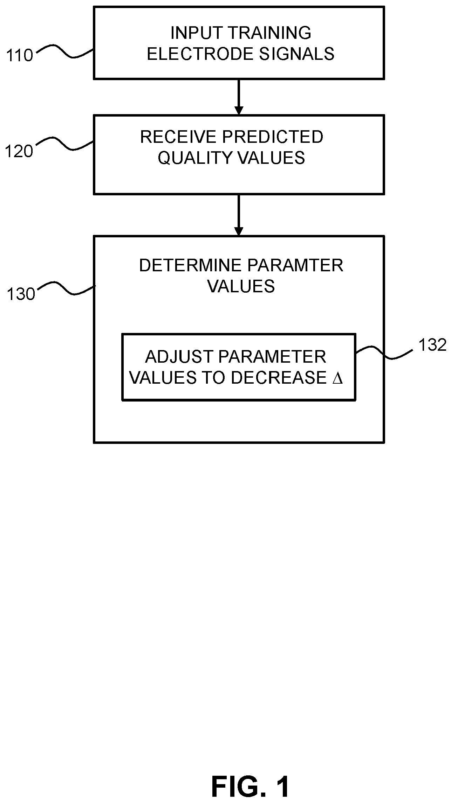

By way of example, a first simplified embodiment will now be described with reference to Fig. 1.

Fig. 1 is a simplified flow diagram of a method for generating a prediction model according to an embodiment. The prediction model is configured to predict a quality value of an electrode configuration (i.e. positional arrangement or relative positioning of the electrodes) for electrophysiological measurements. Specifically, in this example, the prediction model employs a support vector machine (e.g. for classification) and support vector regression (e.g. for regression). The support vector regression provides a quality score that can again be used for classification and decision making (e.g. via comparison of the quality score with a threshold value).

The method begins with step 110 of inputting (to the prediction model) a plurality of training electrode signals for a plurality of known electrode configurations. The training electrode are associated with a plurality of known quality values. That is, training data comprising inputs having known associated outputs is provided to the prediction model.

By way of example, the plurality of training electrode signals may comprise training electrode signals with the one or more signal variables. For instance, the one or more signal variables may include kurtosis of channels, skewness of channels, variance of channels, correlation between channels, mutual Information between channels, spectral coherence between channels, entropy of channels, or cross-entropy between channels. The prediction model may thus be configured to represent an interaction between the one or more signal variables and the plurality of known quality values.

Also, the quality value(s) may comprise at least one of: an F-score; root mean squared error; area under the receiver operating characteristic curve; accuracy; sensitivity; specificity; positive predictive value; and correlation coefficient. That is, various measures of quality may be employed.

In step 120, responsive to the input(s), a plurality of predicted quality values is received from the prediction model. The predicted quality values are generated by the prediction model based on the input training electrode signals, and thus the difference(s) between the output predicted quality values and the known quality values provide an indication of accuracy of the prediction model and/or identify how the prediction model may need to be modified in order to generate more accurate predictions.

Accordingly, in step 130, parameter values of the prediction model are determined based on a difference between the plurality of known quality values and the plurality of predicted quality values. Specifically, in this exemplary embodiment, step 130 of determining parameter values of the prediction model comprises step 132 of adjusting parameter values of the prediction model so as to decrease a difference between: a known quality value associated with training electrode signals for a known electrode configuration; and a predicted quality value received from the prediction model for the known electrode configuration. In this way, an iterative-based training/learning algorithm may be employed to determine the optimal parameter values.

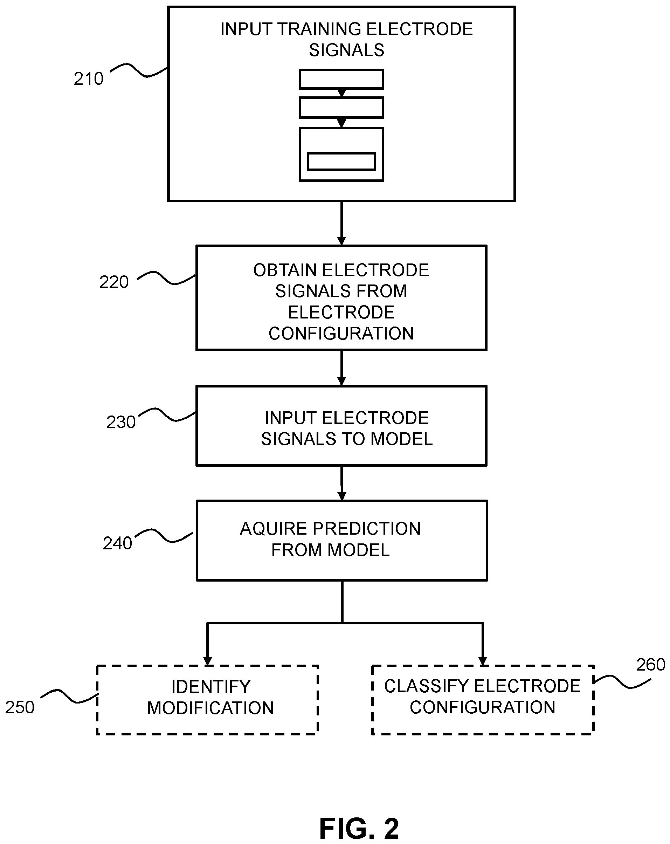

By way of further example, an exemplary embodiment of a method for processing electrode signals from an electrode configuration for electrophysiological measurements will now be described with reference to Fig. 2. In this example, a prediction model generated in accordance with a proposed embodiment is leveraged to acquire a prediction of a quality value of the electrode configuration. That is, a proposed prediction model (such as that generated in the embodiment of Fig. 1) may be used to determine a quality value of an electrode configuration.

The method begins with step 210 of generating a prediction model according to a proposed embodiment. Here, as depicted in Fig. 2, step 210 comprises the method of Fig. 1. The result of step 210 is thus the provision of a prediction model for predicting a quality value of an electrode configuration for electrophysiological measurements.

Next, in step 220, electrode signals from an electrode configuration are obtained and the obtained electrode signals are then input to prediction model in step 230.

In step 240, a prediction of a quality value of the electrode configuration is acquired from the prediction model. The prediction of a quality value is generated by the prediction model based on the inputted electrode signals.

In the example embodiment of Fig. 2, the predicted quality value may be used in additional step 250 and/or 260. Step 250 comprises identifying a modification to the electrode configuration based on the predicted quality value of the electrode configuration. Such a modification may be communicated to an external system and/or user as a suggestion for improving the electrode configuration (e.g. to improve signal accuracy). Step 260 comprises classifying the electrode configuration into one of a plurality of classifications based on the predicted quality value of the electrode configuration. For example, step 260 of classifying the electrode configuration may comprise: comparing the predicted quality value of the electrode configuration against at least one threshold value; and classifying the electrode configuration into one of a plurality of classifications based on the comparison result. Although classification of the electrode configuration has been detailed, alternative embodiments may employ a regressor where the electrode configuration is assessed using a score, for example.

By way of further example and explanation, application of the proposed concept(s) in relation to predicting a quality value for an electrode configuration for fHR extraction will now be discussed.

A synthetic training dataset has been generated by a publically FECGSYN simulator (currently detailed the following internet address: http://fernandoandreotti.github.io/fecgsyn/). This simulator allows for mixing sources related to fetal and maternal cardiac activity, along with muscle activity, electronic noise, and volume conductor. Fetal position (e.g., cephalic or podalic) is also accounted for.

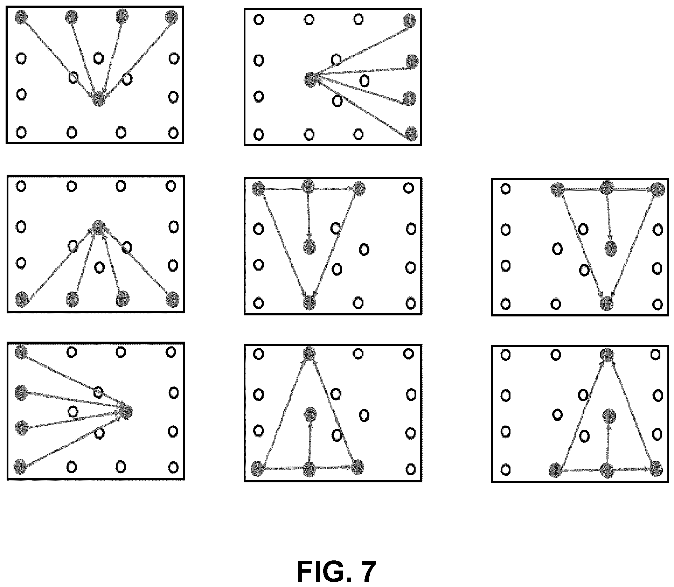

Simulations with sixteen electrodes were performed to generate a suitable dataset to train and test a predictive model. In total, two hundred subjects were simulated and for each of them eight electrode configurations evaluated. The configurations evaluated are presented below with reference to Fig. 7. The quality value of signal-to-noise ratio (SNR), with noise representing all the signal components other than the fetal ECG signal, was less than -20 dB, which is comparable with real acquisitions.

A large set of features has been tested and the prediction models have been employed for feature selection. For each electrode configuration, a known algorithm proposed by Varanini et al. ("A multi-step approach for non-invasive fetal ECG analysis" Computing in Cardiology 2013 (pp. 281-284)) was employed for the estimation of the fetal R-peaks.

From the resulting estimation, two indices were calculated to assess the estimation quality, namely the F-score and the root mean squared error (RMSE). These indices evaluate the ability to detect the R-peaks (F-score) as well as the timing error of the detected R-peaks (RMSE) with respect of the ground truth. A good fetal ECG estimation will show high F-score and low RMSE. SVR can be trained to predict these individual indices or on an analytic combination of the them, such as RMSE/F-score. SVM can be trained to predict directly whether a configuration is sufficiently good (binary classification), corresponding e.g. to RMSE < 50 ms and F-score > 90%. Such a threshold, or alternative thresholds based on a combination of the two indices, can also be applied to the SVR prediction to obtain (binary) classification and discriminate between good- and poor-quality electrode configurations. Binary classification may be preferable for predict good or poor results for a given electrode configuration.

Both for SVM and for SVR, forward 10-fold cross-validation is a viable option for feature selection.

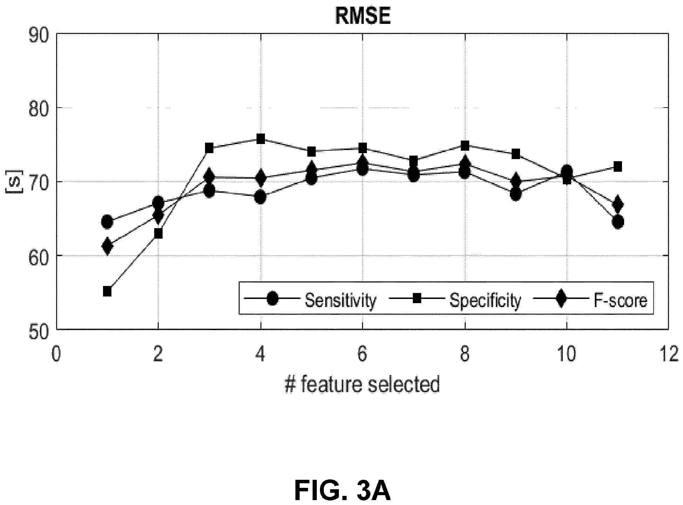

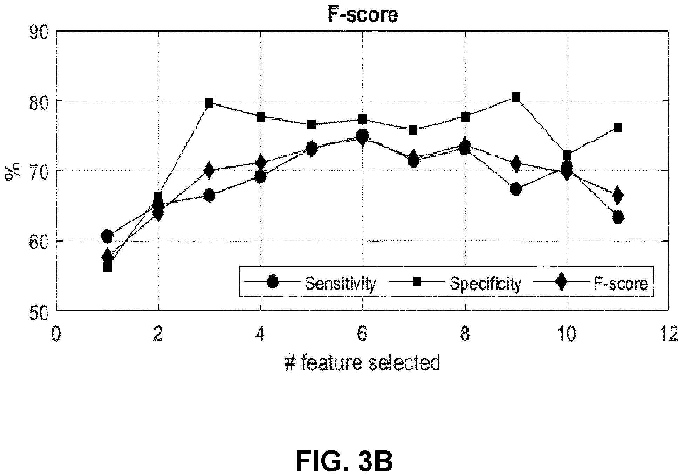

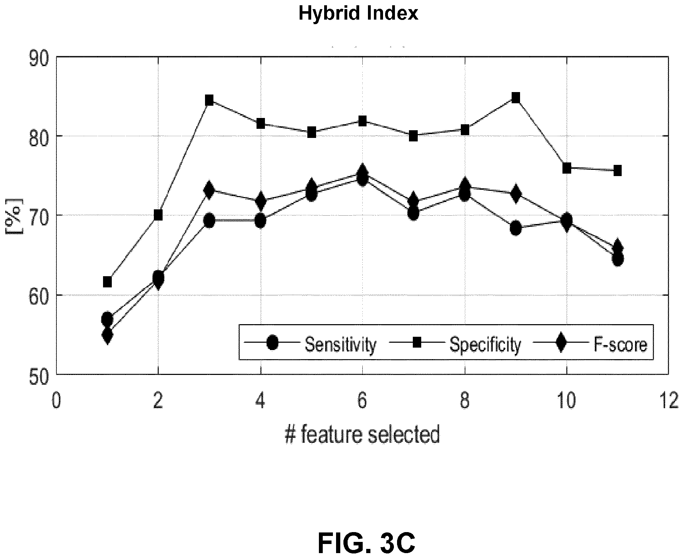

Figs. 3A-3C show the performance of SVM classification in terms of sensitivity, specificity, and F-score of the class (good or poor configuration) prediction for increasing number of features, using either the F-score or the RMSE as separate indices (Figs. 3A & 3B) or their combination (Fig. 3C).

Specifically, Fig. 3A is graph depicting the performance in a test set- against number of selected features, wherein the classifier was trained using the RMSE index. Fig. 3B is graph depicting the performance in a test set against number of selected features, wherein the classifier is trained using the F-score index. Fig. 3C is graph depicting the performance in a test set against number of selected features, wherein the classifier is trained using a hybrid (i.e. combination) of the RMSE and F-Score. In each graph of Figs. 3A-3C, sensitivity, specificity and F-score of the classification is provided for each number of features.

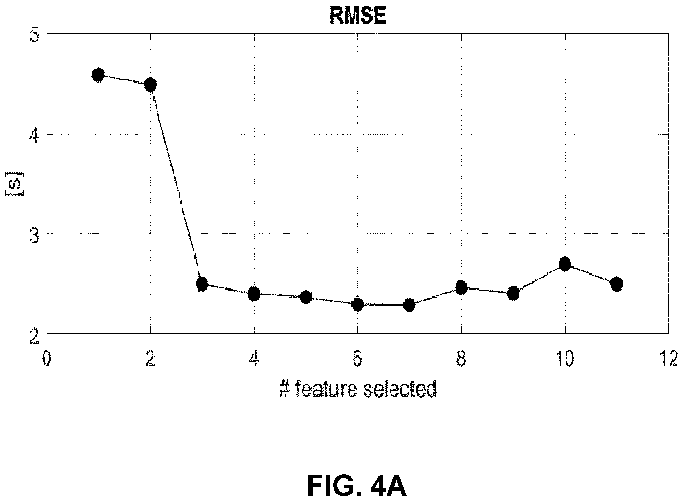

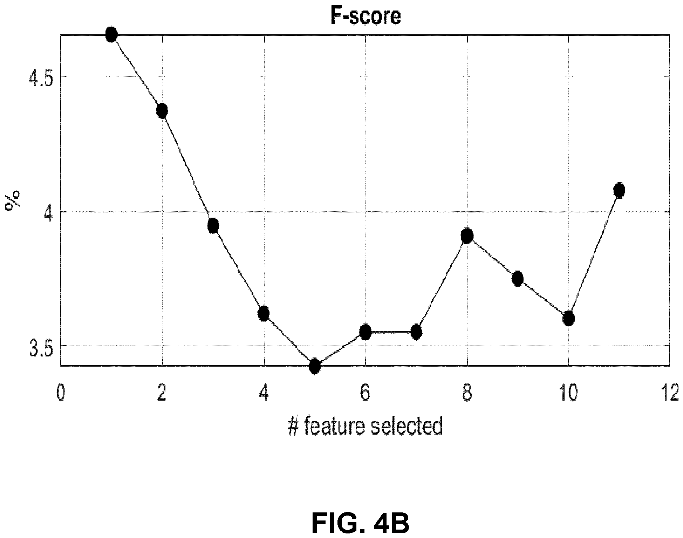

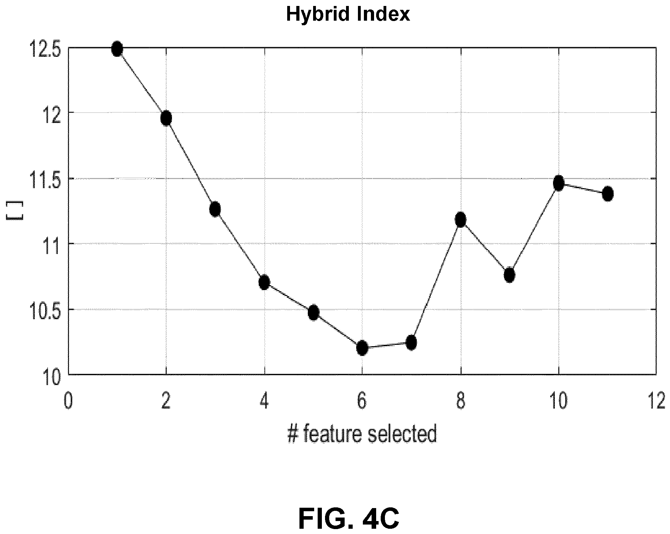

Figs. 4A-4C show the performance of SVR prediction of the resulting F-score and RMSE separately (Figs. 4A & 4B) or their combination (Fig. 4C). The SVR performance is evaluated in terms of prediction error with respect to the real value.

Specifically, Fig. 4A is a graph depicting SVR prediction error in a test set against number of selected features, wherein the regression model is trained using the RMSE index. Fig. 4B is a graph depicting SVR prediction error in a test set against number of selected features, wherein the regression model is trained using F-score index. Fig. 4C is a graph depicting SVR prediction error in a test set against number of selected features, wherein the regression model is trained using a hybrid (i.e. combination) of the RMSE and F-score). In each graph of Figs. 4A-4C, the prediction error is provided for each number of features

Based on the obtained results, a good compromise in order to limit the feature set and achieve good prediction is given by the following set of signal variables:
kurtosis of the channels;
skewness of the channels;
variance of the channels;
correlation between channels;
mutual Information between channels; and
spectral Coherence between channels.

Additional features related to channel entropy and cross-entropy among the channels, as well as other measures of connectivity, may also be employed.

Accordingly, purely by way of example, embodiments may provide a method and/or system for determining an optimal electrode configuration in multichannel electrophysiological monitoring. Such a method includes the step of deriving one or more of the following signal variables from the raw electrode signals (i.e. recorded signal comprising a mixture of a wanted signal (e.g. fetal ECG) and unwanted signal (e.g. mother's ECG, muscle EMG, etc.) and noise), recorded by an arbitrary set of channels of an electrode configuration:
kurtosis of the channels,
skewness of the channels,
variance of the channels,
correlation between channels,
mutual information between channels,
spectral coherence between channels,
entropy and similar correntropy.

Using the derived variable(s) as the input(s) to a proposed prediction model for predicting a quality value of the electrode configuration. This may provide an indication of electrode configuration quality (e.g. accuracy or reliability) prior to actual acquisition of electrophysiological measurement(s), e.g. desired ECG signal.

Two exemplary applications of the proposed concept(s) will now be described in relation to fetal ECG analysis.

### Example 1

In this example, it is assumed that the number of electrodes is limited to the minimum number that still enables performing reliable fetal ECG analysis. Here, the propose concept(s) may be used to guide an operator or clinician to the improved/correct positioning of the electrodes.

For example, a visible and/or audible output signal may be provided to indicate if the electrode configuration meets accuracy/reliability requirements and/or suggest one or more modifications to the electrode configuration, thus supporting proper fetal ECG acquisition. Based on such output signal(s), an operator may reposition the electrodes until positive feedback (e.g. predetermined output signal) is provided by the implemented prediction model (i.e. trained classifier). To assist the operator, guidance may be provided to initiate and/or assist the electrode positioning process.

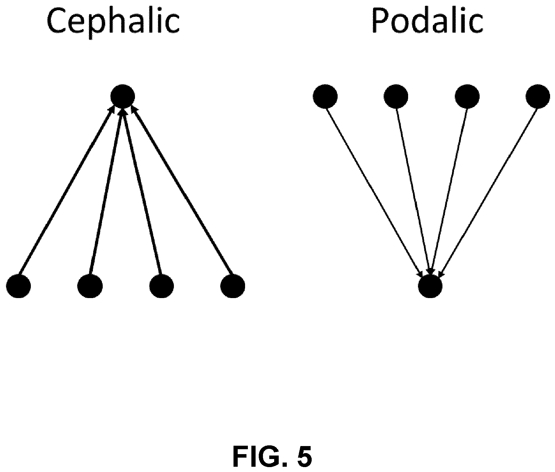

Referring to Fig. 5, there are illustrated preferred electrode configurations for cephalic and podalic presentation of the fetus using five electrodes (as predicted by a prediction model according to an embodiment). The five electrodes are positioned on the mother's abdomen. The arrows indicate the resulting four differential channels.
As can be seen from the electrode configurations depicted in Fig. 5, simulations indicate that a preferred (e.g. optimal) electrode configuration may differ depending on, for example the cephalic or podalic presentation of the fetus.

### Example 2

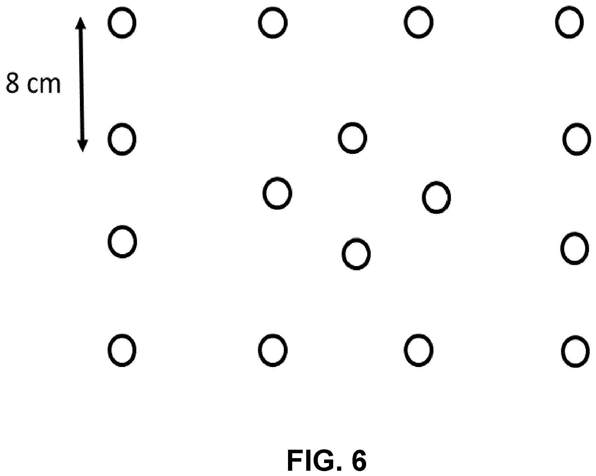

In this example, a larger number of electrodes is considered. Fig. 6 illustrates an electrode grid denoting a set of sixteen possible electrode positions, wherein spacing between adjacent electrode positions is 8 cm. This set of potential electrode positions thus enables multiple electrode configurations involving less than sixteen electrodes.

Assuming the various potential electrode positions shown in Fig. 6, regular tests of a plurality of different electrode configurations may be performed. This may lead to the most reliable electrode configuration for analysis of fetal ECG.

Fig. 7 shows eight different 5-electrode configurations for the electrode grid of Fig. 6. With five electrodes in each electrode configurations, four channels are provided.

When the electronics for signal conditioning is limited to few electrodes, a multiplexer may be employed to switch between the different electrode configurations to be tested. The configuration that results in the best prediction may then be selected to analyze the fetal ECG until the following test is performed. This way, changing (e.g. deterioration) of the fetal ECG analysis due to fetal movement or varying electrode impedance can be overcome (e.g. by dynamically identifying an optimal electrode configuration from obtained predictions).

From the above description of the proposed concept(s) and various exemplary embodiments, it will be understood that various benefits and/or advantages may be provided.

For example, an optimal/preferred combination of channels may be pre-selected and/or electrode repositioning may be guided prior to use (e.g. without any injection of current, without use of dedicated sensors, or without requiring extraction of vital signs).

Also, signal variables (e.g. Kurtosis, Skewness, Variance, Correlation, Mutual Information, Spectral Coherence, entropy and cross-entropy as well as other measures of connectivity) may be calculated as initial step to detect/extract desired signal measurement (e.g. ECG or heart rate) from electrophysiological signals without requiring preprocessing (e.g. noise removal, cross-talk compensation, etc.).

Accordingly, it will be understood that the proposed concept(s) may facilitate electrode configuration assessment and/or recommendation. Embodiments may thus provide for personalized (i.e. user-specific) electrode configuration recommendations, wherein the recommendations are based on information from a prediction model.

Proposed methods and systems (including those of Figs. 1-2) may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices.

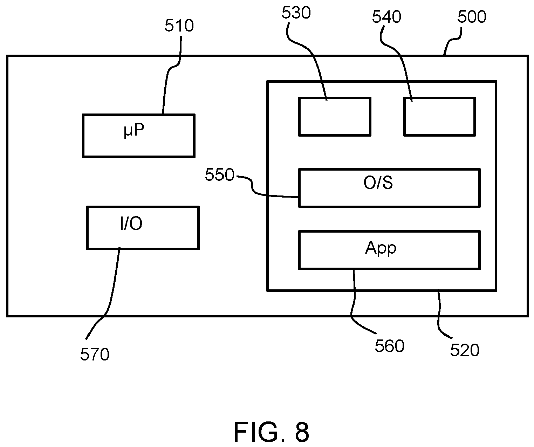

Fig. 8 illustrates an example of a computer 500 within which one or more parts of an embodiment may be employed. Various operations discussed above may utilize the capabilities of the computer 500. For example, one or more parts of a system for processing electrode signals from an electrode configuration for electrophysiological measurements using a prediction model may be incorporated in any element, module, application, and/or component discussed herein. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM. Various storage media may be fixed within a computing device or may be transportable, such that the one or more programs stored thereon can be loaded into a processor.

All of part of a schematic model according to an embodiment may be stored on a storage medium. Data according to an embodiment may be stored on the same storage medium or a different storage medium. The schematic model and/or data may be transmitted as a signal modulated onto an electromagnetic carrier wave. The signal may be defined according to a standard for digital communications. The carrier wave may be an optical carrier, a radio-frequency wave, a millimeter wave, or a near field communications wave. It may be wired or wireless.

To the extent that an embodiment is implemented partly or wholly in hardware, the blocks shown in the diagram(s) may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), a field-programmable gate array (FPGA), and the like; a user input device includes one or more of a mouse, a keyboard, a touch screen display, one or more buttons, one or more switches, one or more toggles, and the like; a display device includes one or more of a liquid crystal display (LCD), an light emitting diode (LED) display, a plasma display, a projection display, a touch screen display, and the like; and databases include one or more memories.

The invention has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

It will also be understood that the disclosed methods may be computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

A proposed system may make use of a processor to perform data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A method of generating a prediction model configured to predict a quality value of an electrode configuration for electrophysiological measurements, the method comprising:
inputting (110) a plurality of training electrode signals for a plurality of known electrode configurations, and with a plurality of known quality values to the prediction model;
receiving (120) a plurality of predicted quality values from the prediction model; and
determining (130) parameter values of the prediction model based on a difference between the plurality of known quality values and the plurality of predicted quality values.

2. The method of claim 1, wherein the prediction model uses a classifier or regressor.

3. The method of claim 1 or 2, wherein the plurality of training electrode signals comprises training electrode signals with the one or more signal variables,
and wherein the prediction model is configured to represent an interaction between with the one or more signal variables and the plurality of known quality values.

4. The method of claim 3, wherein the one or more signal variables include kurtosis of channels, skewness of channels, variance of channels, correlation between channels, mutual Information between channels, spectral coherence between channels, entropy of channels, or cross-entropy between channels.

5. The method of any of claims 1 to 4, wherein the quality value comprises at least one of: an F-score; root mean squared error; area under the receiver operating characteristic curve; accuracy; sensitivity; specificity; positive predictive value; and correlation coefficient.

6. The method of any of claims 1 to 5, wherein the determining (130) parameter values of the prediction model comprises:
adjusting (132) parameter values of the prediction model so as to decrease a difference between: a known quality value associated with training electrode signals for a known electrode configuration; and a predicted quality value received from the prediction model for the known electrode configuration.

7. A method for processing electrode signals from an electrode configuration for electrophysiological measurements, the method comprising:
generating (210) a prediction model according to any of claims 1 to 6;
obtaining (220) electrode signals from an electrode configuration for obtaining electrophysiological measurements of a subject; and
obtaining (240) a prediction of a quality value of the electrode configuration based on inputting the electrode signals to the generated prediction model.

8. The method of claim 7, further comprising:
identifying (250) a modification to the electrode configuration based on the predicted quality value of the electrode configuration

9. The method of claim 7 or 8, further comprising:
classifying (260) the electrode configuration into one of a plurality of classifications based on the predicted quality value of the electrode configuration

10. The method of claim 9, wherein classifying the electrode configuration comprises:
comparing the predicted quality value of the electrode configuration against at least one threshold value; and
classifying the electrode configuration into one of a plurality of classifications based on the comparison result.

11. A method for processing electrode signals from an electrode configuration for electrophysiological measurements using a prediction model configured to predict a quality value of an electrode configuration, the method comprising:
obtaining electrode signals from an electrode configuration for obtaining electrophysiological measurements of a subject; and
acquiring a prediction of a quality value of the electrode configuration based on inputting the electrode signals to the generated prediction model.

12. The method of claim 11, wherein the quality value comprises at least one of: an F-score; root mean squared error; area under the receiver operating characteristic curve; accuracy; sensitivity; specificity; positive predictive value; and correlation coefficient.

13. The method of claim 11 or 12, further comprising:
identifying a modification to the electrode configuration based on the predicted quality value of the electrode configuration

14. The method of any of claims 11 to 13, further comprising:
classifying the electrode configuration into one of a plurality of classifications based on the predicted quality value of the electrode configuration

15. A system for processing electrode signals from an electrode configuration for electrophysiological measurements using a prediction model configured to predict a quality value of an electrode configuration, the system comprising:
a modelling unit (520) configured to generate a prediction model configured to predict a quality value of an electrode configuration for electrophysiological measurements;
an interface (570) configured to obtain electrode signals from an electrode configuration; and
a processing unit (510)configured to acquire a prediction of a quality value of the electrode configuration based on inputting the electrode signals to the generated prediction model.
